# EUROPÄISCHE PATENTSCHRIFT

(11) **EP 2 002 796 B1**
(45) Veröffentlichungstag und Bekanntmachung des Hinweises auf die Patenterteilung: **08.08.2012**
(21) Anmeldenummer: 07110358.4
(22) Anmeldetag: 15.06.2007
(51) Int. Cl.: A61B 19/00

(54) **Computergestütztes Planungsverfahren für die Korrektur von Gelenkknochen-Formveränderungen**
Computer-assisted planning method for correcting changes to the shape of bones in joints
Procédé de planification piloté par informatique pour la correction de modifications de formes d'articulations osseuses

(43) Veröffentlichungstag der Anmeldung: 17.12.2008
(73) Patentinhaber: BrainLAB AG, 85622 Feldkirchen (DE)
(72) Erfinder: Tuma, Gregor, 81675, München (DE); Schindler, Florian, 80937, München (DE); Fricke, Marc, 85622, Feldkirchen (DE); Haimerl, Martin, 82205, Gilching (DE)
(74) Vertreter: Schwabe - Sandmair - Marx

(56) Entgegenhaltungen:
- WO-A-01/35842
- DE-A1- 10 057 023
- US-A- 5 871 018
- US-A1- 2006 241 388
- US-B1- 6 711 432

## Beschreibung

Die Erfindung betrifft ein computergestütztes Planungsverfahren für die Korrektur von Gelenkknochen-Formveränderungen. Gelenkknochen-Formveränderungen sind eine mögliche Ursache für arthritische Gelenkkrankheiten. Beispielsweise können im Bereich des Beckengelenks Knochenanomalien auftreten, die bei der Beinbewegung zu Knochen-Kollisionen im Gelenkbereich führen und - insbesondere auf längere Dauer - Abnutzungsbeschwerden hervorrufen. Zur Vorbereitung von Rekonstruktions-Behandlungen, also solchen Behandlungen, welche wieder eine geeignete Knochenform herstellen, ist eine Planung notwendig, die computergestützt erfolgen kann.

Die Grundlage solcher Planungen war bisher die Bestimmung der Kontur von Gelenkknochen auf zweidimensionaler Basis, beispielsweise in einer bestimmten Schnittebene. Im "Journal of Bone and Joint Surgery", Volume 84-B, No. 4, May 2002, Seiten 556 bis 560, beschreiben Nötzli et al die Bestimmung der Kontur eines Oberschenkelhalses, und hierfür wird ein Mess- bzw. Winkelsystem angegeben, auf welches auch hierin Bezug genommen wird.

Ein weiteres Planungsverfahren wird in DE10057032 offenbart.

Es ist die Aufgabe der vorliegenden Erfindung, ein computergestütztes Planungsverfahren für die Korrektur von Gelenkknochen-Formveränderungen bereitzustellen, welches spätere, eventuell navigationsgestützte Behandlungen derart unterstützt, dass eine optimale Rekonstruktion des Knochens erzielt werden kann.

Diese Aufgabe wird erfindungsgemäß durch ein Verfahren gemäß dem Anspruch 1 gelöst. Die Unteransprüche definieren bevorzugte Ausführungsformen der Erfindung.

Das computergestützte Planungsverfahren für die Korrektur von Gelenkknochen-Formveränderungen gemäß der vorliegenden Erfindung umfasst die folgenden Schritte:
- ein dreidimensionaler Abbildungs-Datensatz, speziell ein Volumenbilddatensatz der Gelenkknochenumgebung wird bereitgestellt, insbesondere mittels eines medizintechnischen, bildgebenden Verfahrens erstellt;
- die Gelenkknochenelemente werden im Datensatz identifiziert und der Form nach bestimmt, insbesondere mittels eines computergestützten Bildsegmentierungsverfahrens;
- mittels einer graphischen Datenverarbeitung wird dem zu rekonstruierenden Abschnitt des Gelenkknochens eine zuordnungsfähige, insbesondere geometrische Grundform einbeschrieben;
- Abweichungen des Gelenkknochens von der Grundform werden durch die Feststellung von Konturabständen zwischen der Grundform und der Gelenkknochenform in unterschiedlichen Ebenen bestimmt, wobei ein dreidimensionales Abweichungsvolumen ermittelt wird;
- das Abweichungsvolumen wird der Korrekturplanung zugrunde gelegt.

Mit anderen Worten wird gemäß der Erfindung bei der Planung nicht nur in zweidimensionaler Weise eine Konturabweichung festgestellt, sondern das zu entfernende bzw. von einer gewünschten Form abweichende Knochenteil wird volumenmäßig, also dreidimensional erfasst. Der Vorteil liegt dabei insbesondere darin, dass ein solches Volumen einerseits im computergestützten Planungssystem als Bild gut separat oder im Verhältnis zum Knochen ausgegeben werden kann, so dass bei dem Behandelnden schon ein dreidimensionaler Eindruck dessen entsteht, was während der Behandlung entfernt werden muss. Andererseits können weitere Erwägungen hinsichtlich des zu entfernenden Knochenvolumens angestellt werden, wenn das erfindungsgemäße Ergebnis vorliegt, also das dreidimensionale Abweichungsvolumen. Es ist nämlich möglich, bestimmte Stellen- oder Teilvolumina von der Rekonstruktion auszunehmen oder als nicht zu entfernende Volumina zu kennzeichnen, wenn andere Aspekte für die optimale Rekonstruktion eine gewisse Priorität haben. Hierauf wird später noch detailliert eingegangen.

Das bildgebende Verfahren kann ein Computertomographieverfahren, ein Kernspintomographieverfahren oder ein Röntgenverfahren mit Volumenerfassung sein. Es kann aber jede mögliche 3D-Modellerzeugung der Gelenkstruktur verwendet werden. Dies kann evtl. auch ein Scannen des Objekts mit anderen Verfahren beinhalten (z.B. Oberflächenscan).

Es besteht im Rahmen der vorliegenden Erfindung die Möglichkeit, das Abweichungsvolumen durch den Vergleich der Konturen in um eine Achse gedrehten Schnittebenen zu bestimmen, wobei die Achse eine für den Gelenkknochen charakteristische Achse ist. Andererseits ist die Erfindung hierauf nicht eingeschränkt; das Abweichungsvolumen kann z.B. auch durch den Vergleich der Konturen in nebeneinander liegenden Schnittebenen bestimmt werden.

Die einbeschriebene Grundform kann eine Kugelform, eine Sattelform, eine Zylinderform oder eine Kombination aus solchen Formen sein, und sie wird je nach dem Anwendungsfall auszuwählen sein. Eine weitere Möglichkeit besteht darin, eine insbesondere in der Größe angepasste Grundform für den Gelenkknochen aus einem anatomischen Atlas, einem generischen oder statistischen Modell zu verwenden. Der in Frage kommende Gelenkknochen kann der Oberschenkelhalsknochen sein, wobei die Grundform dann vorzugsweise eine in den Oberschenkelknochen-Kopf einbeschriebene Kugelform umfasst.

Bei einer Ausführungsvariante werden die Konturabweichungen von der Kugelform sukzessiv in mehreren Schnittebenen bestimmt, die um die Oberschenkelhalsachse verdreht sind. Dabei besteht die Möglichkeit, die Konturabweichungen in einer oder jeder Schnittebene entlang eines Radiusvektors der Kugel zu bestimmen, der über den Messbereich zu der Halsachse des Oberschenkelhalses sich verändernde Winkel (α) einnimmt. Der Messbereich bezüglich des genannten Winkels (α) (vgl. Publikation von Noetzli et al) zum freien Oberschenkelkopf-Ende hin beginnt dort, wo die Kopfkontur erstmals von der Grundform abweicht. Bei solchen Ausgestaltungen kann dann der Messbereich bezüglich des Winkels (α) zum Oberschenkelhals hin dort enden, wo der Winkel (α) einen ermittelten Standardwert annimmt. Die Bestimmmung des Standardwerts kann anhand einer Auswertung standardisierter Modelle (z.B. generische oder statistische Formmodelle) bestimmt werden. Der Standardwert kann je nach Ausrichtung der Schnittebene variieren. Alternativ kann der Messbereich dort enden, wo der Winkel (α) einen Wert annimmt, der einem gespiegelten Winkel (β) entspricht, wobei der Winkel (β) der Winkel ist, den ein Kugel-Radiusvektor zur Halsachse (g) einnimmt, wenn er auf der Seite, die der Konturabweichung gegenüberliegt, auf den Übergang zwischen Oberschenkelknochen-Kopf und Oberschenkelhals zeigt.

Die folgenden erhaltenen Daten über das Abweichungsvolumen können einzeln oder in Kombination von einer verwendeten computergestützten Datenverarbeitungseinrichtung zur weiteren Nutzung oder Verarbeitung ausgegeben werden:
- Schnittebenen mit Konturabweichungen und/oder Grundform, speziell die Schnittebene mit der größten Konturabweichung;
- ermittelte Messbereichswinkel (α, β);
- Knochencharakteristika wie Gelenkdrehzentrum, Halsachsenlage, Beckenebenen, Oberschenkelknochen-Achsenlage.

Mit den Daten kann in verschiedener Weise verfahren werden; man kann sie unter anderem auf einer Anzeigeeinrichtung, insbesondere einem Bildschirm ausgeben und/oder an ein medizintechnisches Navigationssystem ausgeben.

Bei einer Ausführungsform des erfindungsgemäßen Verfahrens wird aus dem Abweichungsvolumen ein Rekonstruktionsvolumen berechnet, und zwar unter der Inbetrachtziehung einer oder mehrer der folgenden Randbedingungen:
- eine ausreichende Knochentiefe muss erhalten bleiben;
- eine neue Gelenkknochenform muss glatte Übergänge und soll keine oder minimale Einbuchtungen haben;
- eine neue Gelenkknochenform sollte möglichst nahe an eine natürliche Gelenkknochenform heranreichen (Vergleich mit Standardformen);
- der Einfluss von Knochen-Kollisionsbereichen wird je nach seinen kritischen Eigenschaften gewichtet;
- das Rekonstruktionsvolumen, speziell das zu entfernende Volumen sollte möglichst minimiert werden.

Es besteht erfindungsgemäß ferner die Möglichkeit, ein Verfahren so durchzuführen, dass eine neue Gelenkknochenform mit Hilfe des Abweichungsvolumens bzw. des Rekonstruktionsvolumens bestimmt wird und mit der neuen Gelenkknochenform computergestützt auf der Basis einer Kollisionsdetektion eine Gelenkbewegungssimulation durchgeführt wird. Dabei kann die zu erwartende Vergrößerung des Bewegungsspielraums ermittelt und insbesondere auch ausgegeben werden.

Die Erfindung wird im Weiteren unter Bezugnahme auf die beiliegenden Zeichnungen näher erläutert. Sie kann alle hierin beschriebenen Merkmale einzeln sowie in jedweder sinnvollen Kombination umfassen. In den Zeichnungen zeigen:
- Figur 1: eine Schnittzeichnung durch den Oberschenkelhals-Bereich mit eingezeichneten Parametern für die Rekonstruktionsplanung; und
- Figur 2: eine Darstellung der Schnittebenen, die für die Bestimmung des Abweichungsvolumens verwendet werden.

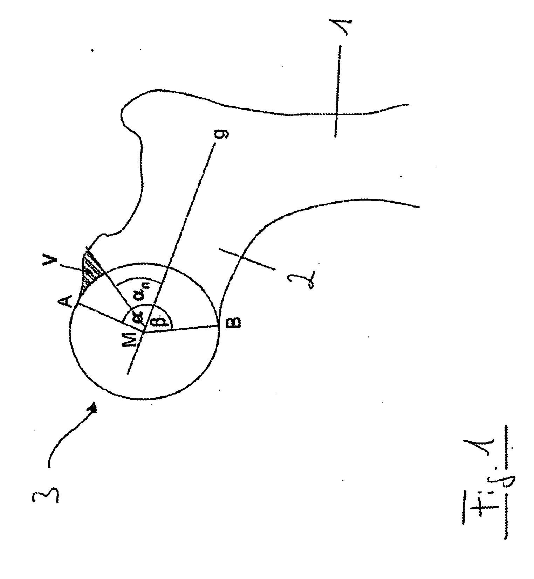

In Figur 1 ist der Halsbereich eines Oberschenkelknochens 1 gezeigt, mit dem Schenkelhals 2 und dem Gelenkkopf 3. Dem Gelenkkopf 3 ist eine Kugel einbeschrieben, die in der Schnittdarstellung der Figur 1 als Kreis abgebildet wird. Der Mittelpunkt der Kugel ist mit M bezeichnet und bildet gleichzeitig den Mittelpunkt des Gelenkkopfes (Femurkopf). Mit dem Bezugszeichen g ist die Halsachse (Neckachse) bezeichnet, und der schraffierte Bereich V markiert überstehendes Knochenvolumen, welches im Hüftgelenk Kollisionen und hierdurch hervorgerufene arthritische Krankheiten hervorrufen kann.

Um das Abweichungsvolumen V zu bestimmen, werden bestimmte Winkel verwendet, die in Figur 1 in dem Kreis um den Mittelpunkt M eingetragen sind. Hierbei bezeichnet der Winkel α den Winkel zur Halsachse g, bei dem die Kontur des Knochens erstmalig von der Kugelform abweicht, und der Winkel αₙ bezeichnet den gewünschten Winkel α, bei dem die Kontur eigentlich erstmalig von der Kugelform abweichen sollte. Der von diesen beiden Winkeln eingefasste Bereich des Knochens außerhalb des Kreises (einbeschriebene Kugel) bildet das Abweichungsvolumen V.

Der Winkel β ist der Winkel auf der unteren ("gesunden") Seite, des Schenkelhalses, bei dem die Kontur erstmalig von der Kreiskontur abweicht.

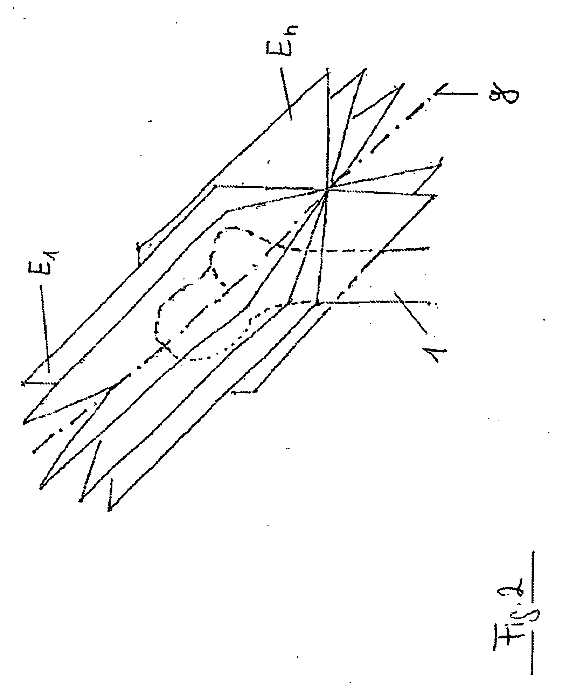

Die Figur 2 zeigt, wie das Abweichungsvolumen dreidimensional und automatisch bestimmt wird. Es werden dabei computergestützt aus dem Volumen-Bilddatensatz (CT, MR, ...) die Schenkelhalsachse g des Oberschenkelknochens 1 und eine ausreichende Anzahl von um diese Achse g verdrehten Ebenen E₁ bis Eₙ bestimmt. Für jede Ebenenrekonstruktion, die in einem Winkel um die Halsachse g gedreht ist, wird eine Analyse der Kreisförmigkeit des Oberschenkelhals-Kopfes mit den in Figur 1 dargestellten Parametern gemacht. In jeder Ebenenrekonstruktion wird also der Winkel gemessen, der zwischen der Halsachse und dem Punkt auf dem einbeschriebenen Kreis liegt, wo die Knochenoberfläche die Kreisform verlässt. Die Ebenen mit dem maximalen Unterschied zwischen den Winkeln ist die Ebene, welche verwendet werden kann, um das Krankheitsbild des Patienten darzustellen und eine Patienten-Vergleichsmessung zu machen. Idealerweise ist diese Ebene eine der vorgegebenen Rekonstruktionsansichten in der Darstellungsoption der verwendeten Software. Wie schon erwähnt, kann der Winkel β zwischen der Halsachse g und der Strecke M-B als Zielvorgabe für die Rekonstruktion auf der gegenüberliegenden Seite genutzt werden. Alternativ können auch fest vorgegebene Standardwerte dafür verwendet werden, die aus einem generischen oder statistischen Modell erzeugt werden können.

Wenn diese Berechnungen für jede Rekonstruktion bzw. jede Rekonstruktionsebene gemacht werden, kann eine vorgeschlagene Konturgrenze ermittelt und dargestellt werden, und die entsprechenden Werte können ferner an ein Navigationssystem zur Unterstützung der Behandlung weitergegeben werden.

Grundsätzlich kann die erfindungsgemäße Rekonstruktions-Planung die folgenden Konzepte zur Grundlage haben:
1. Es wird ein Vorschlag zur Rekonstruktion bzw. Neuformung des Oberschenkelhalses gemacht, der auf dem oben beschriebenen α- und β-Winkelkonzept basiert. Das Konzept wird von dem bisherigen zweidimensionalen Ansatz erfindungsgemäß auf einem dreidimensionalen Ansatz erweitert, d. h. die β-Winkel für alle gedrehten Ebenen werden von der nicht zu behandelnden Referenzseite auf die zu behandelnde Seite gespiegelt, und diese Winkel werden als Minimalgrenze für die Definition des zu entfernenden Volumens benutzt. Alternativ werden Standardwerte, die z.B. anhand eines generischen oder statistischen Formmodells ermittelt werden, als Minimalgrenze für die Definition des zu entfernenden Volumens benutzt.
2. Ein Vorschlag zur Neuformung des Oberschenkelhalses und/oder der Gelenkpfannen-Grenze wird basierend auf dem Konzept der Zurückgewinnung von Bewegungsspielraum gemacht. Dabei wird basierend auf einem Kollisions-Detektionsalgorhitmus eine Bewegungsspielraum-Analyse mit gewünschtem Bewegungsspielraum-Werten (z.B. Standardwerten) erstellt, und alle Bereiche, die zu einer Knochenkollisionbeitragen, werden identifiziert und bei der Rekonstruktion berücksichtigt.
3. Überprüfung des Neuformungs-Vorschlags auf Tiefe basierend auf einem "Schenkelhals-Frakturrisiko-Konzept". Hierbei muss garantiert werden, dass eine gewisse Knochenmenge (z. B. gemäß der Kortikaltiefe) bei der Neuformung stehen bleibt.

Die Planung kann eines dieser Konzepte verwenden oder für eine Kombination der entsprechenden Kriterien optimiert werden. Zusätzlich können weitere Randbedingungen in die Planung mit einbezogen werden, nämlich unter anderem die folgenden:
- Glattheit "des zu entfernenden Volumens" (Abrasionsvolumen) und der verbleibenden Knochenstrukturen; dieses Kriterium sollte sicherstellen, dass keine Vertiefungen in den Knochen eingebracht werden, die zu Frakturen führen könnten.
- Die natürliche Form (z.B. Krümmungen) des Knochens (z. B. gemäß einem standardisierten Knochenmodell) sollte reproduziert werden; Knochenanomalien würden dabei negativ eingewertet.
- Der Einfluss der Kollisionsbereiche kann für die Berechnung des abzunehmenden Volumens gewichtet werden. Die Gewichtung kann eine Schätzung darüber umfassen, wie kritisch die entsprechenden Kollisionsbereiche sein können. Dies kann durch die Position des Bereichs (bsp. unter Verwendung der α-Winkel) oder durch die Konfiguration des Gelenks, wo die Kollisionen auftreten, eingeschätzt werden (z.B. Vergleich von Flexion/Extension, innerer/externer Rotation, Abduktion/Aduktion mit standardisierten Werten für den Bewegungsspielraum).
- Das Volumen, der Bereich oder die Tiefe der Abrasion sollte gemäß bestimmten Randbedingungen minimiert werden. Die Tiefenwerte können gemäß der Zuverlässigkeit und Schwere erfasster Kollisionen oder Knochenanomalien gewichtet werden. Beispielsweise können Tiefenwerte verwendet werden, um die Häufigkeit oder Schwere einer Kollision einzuwerten.

Mit solchen Randbedingungen kann das abzunehmende Volumen bzw. das Rekonstruktionsvolumen auf Bereiche ausgedehnt werden, die weiter von dem Haupt-Kollisionsbereichen entfernt sind, z.B. hin zum Schenkelhals. Diese Randbedingungen werden verwendet, um ein minimales Trauma sowie Stabilität und Glätte des Knochens nach dem Eingriff sicherzustellen.

Ein Verfahrensablauf für die erfindungsgemäße Planung kann die folgenden Schritte umfassen:
1. Segmentierung eines CT-Scans (Abgrenzung von Knochen und Gewebe)
2. Segmentierung des erhaltenen Bildmaterials zur Abgrenzung der Knochen (rechter Oberschenkel, linker Oberschenkel, Becken) und Zuordnung von atlas-basierten Eigenschaften der Knochenelemente (Drehzentrum, Halsachse, Femurachse, Beckenebenen);
3. Prüfung des Resultats und eventuell Optimierung der Segmentierung sowie der Zuordnung der Knochencharakteristika (manuell/automatisch);
4. Automatisches Einbeschreiben einer Grundform (z.B. Kugel) in den Knochen (Gelenkkopf);
5. Analyse der Anomalie-Parameter (α-, β-Winkel) in dreidimensionaler Weise, durch eine zirkuläre Analyse ("Kugelprobe");
6. Prüfung der Tiefe des Knochenvolumens durch eine Vermessung des Abstandes zwischen Halsachse und Oberfläche; Ausgabe des Tiefen-Prozentwertes ("Fraktur-Sicherheitswert");
7. Kollisions-Analyse (z.B. durch Software-Simulation);
8. Ausgabe von Parametern, z. B. Vergrößerung des Bewegungsspielraums, maximale α-, β-Winkel und Knochenvolumen-Vorschlag, wobei vorgegebene Ansichten vorgeschlagen werden, um eine einfache Beurteilung durch den Benutzer zu ermöglichen.

Wenn nach dem Schritt 8. die Information noch ungenügend ist, die Planung nicht erfolgreich war oder weiterer Optimierungsbedarf besteht, kann der Verwender Korrekturen vornehmen und das Verfahren insbesondere beginnend mit dem Schritt 5. noch einmal ablaufen lassen, bis das gewünschte Ergebnis erzielt ist.

## Patentansprüche

1. Computergestütztes Planungsverfahren für die Korrektur von Gelenkknochen-Formveränderungen mit den folgenden Schritten:
- ein dreidimensionaler Abbildungsdatensatz, speziell ein Volumenbilddatensatz der Gelenkknochenumgebung wird bereitgestellt, insbesondere mittels eines medizintechnischen, bildgebenden Verfahrens erstellt;
- die Gelenkknochenelemente (2, 3) werden im Datensatz identifiziert und der Form nach bestimmt, insbesondere mittels eines computergestützten Bildsegmentierungsverfahrens;
- mittels einer graphischen Datenverarbeitung wird dem zu rekonstruierenden Abschnitt des Gelenkknochens (1) eine zuordnungsfähige, insbesondere geometrische Grundform einbeschrieben;
- Abweichungen des Gelenkknochens (1) von der Grundform werden durch die Feststellung von Konturabständen zwischen der Grundform und der Gelenkknochenform in unterschiedlichen Ebenen bestimmt, wobei ein dreidimensionales Abweichungsvolumen (V) ermittelt wird; und
- das Abweichungsvolumen (V) wird der Korrekturplanung zugrunde gelegt.

2. Verfahren nach Anspruch 1, bei dem das bildgebende Verfahren ein Computertomographieverfahren, ein Kemspintomographieverfahren oder ein Röntgenverfahren mit Volumenerfassung ist.

3. Verfahren nach Anspruch 1 oder 2, bei dem das Abweichungsvolumen (V) durch den Vergleich der Konturen in um eine Achse (g) gedrehten Schnittebenen (E₂, Eₙ) bestimmt wird, wobei die Achse (g) eine für den Gelenkknochen (1) charakteristische Achse ist.

4. Verfahren nach einem der Ansprüche 1 bis 3, bei dem das Abweichungsvolumen (V) durch den Vergleich der Konturen in nebeneinander liegenden Schnittebenen (E₂, Eₙ) bestimmt wird.

5. Verfahren nach einem der Ansprüche 1 bis 4, bei dem die Grundform eine Kugelform, eine Sattelform, eine Zylinderform oder eine Kombination aus solchen Formen umfasst.

6. Verfahren nach einem der Ansprüche 1 bis 5, bei dem die Grundform eine, insbesondere in der Größe angepasste, Grundform für den Gelenkknochen (1) aus einem anatomischem Atlas oder einem anderen Vergleichsmodell, insbesondere einem generischen oder statistischen Modell ist.

7. Verfahren nach einem der Ansprüche 1 bis 6, bei dem der Gelenkknochen (1) der Oberschenkelhalsknochen (2) ist und die Grundform eine in den Oberschenkelknochen-Kopf einbeschriebene Kugelform umfasst.

8. Verfahren nach Anspruch 7, bei dem die Konturabweichungen von der Kugelform sukzessive in mehreren Schnittebenen (E₁-Eₙ), die um die Oberschenkelhalsachse (g) verdreht sind, bestimmt werden.

9. Verfahren nach Anspruch 8, bei dem die Konturabweichungen in einer oder jeder Schnittebene (E₂, Eₙ) entlang eines Radiusvektors der Kugel bestimmt werden, der über den Messbereich zu der Halsachse des Oberschenkelhalses (2) sich verändernde Winkel (α) einnimmt.

10. Verfahren nach Anspruch 9, bei dem der Messbereich bezüglich des Winkels (α) zum freien Oberschenkelkopf-Ende hin dort beginnt, wo die Kopfkontur erstmals von der Grundform abweicht.

11. Verfahren nach Anspruch 9 oder 10, bei dem der Messbereich bezüglich des Winkels (α) zum Oberschenkelhals hin dort endet (αₙ), wo der Winkel (α) einen Wert annimmt, der einem gespiegelten Winkel (β) entspricht, wobei der Winkel (β) der Winkel ist, den ein Kugel-Radiusvektor zur Halsachse (g) einnimmt, wenn er auf der Seite, die der Konturabweichung gegenüberliegt, auf den Übergang zwischen Oberschenkelknochen-Kopf und Oberschenkelhals (2) zeigt.

12. Verfahren nach Anspruch 9 oder 10, bei dem der Messbereich bezüglich des Winkels (α) zum Oberschenkelhals (2) hin dort endet (αₙ), wo der Winkel (α) einen Standardwert annimmt, der fest vorgegeben oder aus einem generischen oder statistischen Modell errechnet wird, wobei der Standardwert je nach der Ausrichtung der Schnittebene (E₂, Eₙ) variieren kann.

13. Verfahren nach einem der Ansprüche 1 bis 12, bei dem die folgenden erhaltenen Daten über das Abweichungsvolumen (V), einzeln oder in Kombination, von einer verwendeten computergestützten Datenverarbeitungseinrichtung zur weiteren Nutzung oder Verarbeitung ausgegeben werden:
- Schnittebenen mit Konturabweichungen und/oder Grundform, speziell die Schnittebene mit der größten Konturabweichung;
- ermittelte Messbereichswinkel (α, β);
- Knochencharakteristika wie Gelenkdrehzentrum, Halsachsenlage, Beckenebenen, Oberschenkelknochen-Achsenlage.

14. Verfahren nach Anspruch 13, bei dem die Daten auf einer Anzeigeeinrichtung, insbesondere einem Bildschirm, ausgegeben werden.

15. Verfahren nach Anspruch 13 oder 14, bei dem die Daten an ein medizintechnisches Navigationssystem ausgegeben werden.

16. Verfahren nach einem der Ansprüche 1 bis 15, bei dem aus dem Abweichungsvolumen ein Rekonstruktionsvolumen (V) berechnet wird, und zwar unter Inbetrachtziehung einer oder mehrerer der folgenden Randbedingungen:
- eine ausreichende Knochentiefe muss erhalten bleiben;
- eine neue Gelenkknochenform muss glatte Übergänge und soll keine oder minimale Einbuchtungen haben;
- eine neue Gelenkknochenform sollte möglichst nahe an eine natürliche Gelenkknochenform heranreichen (Vergleich mit Standardformen);
- der Einfluss von Knochen-Kollisionsbereichen wird je nach seinen kritischen Eigenschaften gewichtet;
- das Rekonstruktionsvolumen, speziell das zu entfernende Volumen sollte möglichst minimiert werden.

17. Verfahren nach einem der Ansprüche 1 bis 16, bei dem eine neue Gelenkknochenform mit Hilfe des Abweichungsvolumens (V) bzw. des Rekonstruktionsvolumens bestimmt wird und mit der neuen Gelenkknochenform computergestützt auf der Basis einer Kollisionsdetektion eine Gelenkbewegungssimulation durchgeführt wird, wobei insbesondere die zu erwartende Vergrößerung des Bewegungsspielraums ermittelt und insbesondere auch ausgegeben wird.

18. Programm, das, wenn es auf einem Computer läuft oder in einem Computer geladen ist, den Computer veranlasst, ein Verfahren gemäß einem der Ansprüche 1 bis 17 durchzuführen.

19. Computerprogramm-Speichermedium, das ein Programm nach Anspruch 18 aufweist.

## Claims

1. A computer-assisted planning method for correcting changes in the shape of joint bones, comprising the following steps:
- a three-dimensional image data set - specifically, a volume image data set - of the joint bone environment is provided, in particular produced by means of a medical imaging method;
- the joint bone elements (2, 3) are identified in the data set and determined in terms of their shape, in particular by means of a computer-assisted image segmenting method;
- the portion of the joint bone (1) which is to be reconstructed is inscribed with an assignable base shape, in particular a geometric base shape, by means of graphic data processing;
- deviations of the joint bone (1) from the base shape are determined by ascertaining contour distances between the base shape and the shape of the joint bone in different planes, wherein a three-dimensional deviating volume (V) is ascertained; and
- the deviating volume (V) is used as the basis for correction planning.

2. The method according to claim 1, wherein the imaging method is a computed tomography method, a nuclear spin tomography method or an x-ray method comprising volumetric detection.

3. The method according to claim 1 or 2, wherein the deviating volume (V) is determined by comparing the contours in incision planes (E₂, Eₙ) which are rotated about an axis (g), wherein the axis (g) is one which is characteristic of the joint bone (1).

4. The method according to any one of claims 1 to 3, wherein the deviating volume (V) is determined by comparing the contours in adjacent incision planes (E₂, Eₙ).

5. The method according to any one of claims 1 to 4, wherein the base shape comprises a spherical shape, a saddle shape, a cylindrical shape or a combination of such shapes.

6. The method according to any one of claims 1 to 5, wherein the base shape is a base shape for the oint bone (1) from an anatomical atlas or other comparative model, in particular a generic or statistical model, which is in particular adapted in size.

7. The method according to any one of claims 1 to 6, wherein the joint bone (1) is the femoral neck bone (2), and the base shape comprises a spherical shape which is inscribed into the head of the femoral neck bone.

8. The method according to claim 7, wherein the contour deviations from the spherical shape are determined successively in a plurality of incision planes (E₁ to Eₙ) which are rotated about the femoral neck axis (g).

9. The method according to claim 8, wherein the contour deviations are determined in one or in each incision plane (E₂, Eₙ) along a radius vector of the sphere which assumes varying angles (α) with respect to the neck axis of the femoral neck (2) over the measurement range.

10. The method according to claim 9, wherein the measurement range with respect to the angle (α) towards the free end of the femoral head begins where the contour of the head deviates from the base shape for the first time.

11. The method according to claim 9 or 10, wherein the measurement range with respect to the angle (α) towards the femoral neck ends (αₙ) where the angle (α) assumes a value which corresponds to a mirrored angle (β), wherein the angle (β) is the angle assumed by a spherical radius vector with respect to the neck axis (g) when it points to the transition between the head of the femoral bone and the femoral neck (2) on the side opposite the contour deviation.

12. The method according to claim 9 or 10, wherein the measurement range with respect to the angle (α) towards the femoral neck (2) ends (αₙ) where the angle (α) assumes a standard value which is rigidly preset or is calculated from a generic or statistical model, wherein the standard value can vary depending on the orientation of the incision plane (E₂, Eₙ).

13. The method according to any one of claims 1 to 12, wherein the following obtained data concerning the deviating volume (V) are outputted, individually or in combination, by a computer-assisted data processing means used, for subsequent use or processing:
- incision planes with contour deviations and/or a base shape, specifically the incision plane with the greatest contour deviation;
- ascertained measurement range angles (α, β);
- bone characteristics such as the centre of rotation of the joint, the position of the neck axis, the pelvic planes and the axial position of the femoral bone.

14. The method according to claim 13, wherein the data are outputted on a display means, in particular a screen.

15. The method according to claim 13 or 14, wherein the data are outputted to a medical navigation system.

16. The method according to any one of claims 1 to 15, wherein a reconstructed volume (V) is calculated from the deviating volume, by considering one or more of the following ancillary conditions:
- a sufficient bone depth has to be maintained;
- a new shape of the joint bone must have smooth transitions and should have no indentations or minimal indentations;
- a new shape of the joint bone should come as near as possible to a natural shape of the joint bone (comparison with standard shapes);
- the influence of bone collision regions is weighted depending on its critical properties;
- the reconstructed volume, specifically the volume to be removed, should be minimised as far as possible.

17. The method according to any one of claims 1 to 16, wherein a new shape of the joint bone is determined with the aid of the deviating volume (V) and/or reconstructed volume, and a joint movement is simulated with computer assistance, using the new shape of the joint bone, on the basis of collision detection, wherein in particular the increase in the range of motion to be expected is ascertained and in particular also outputted.

18. A program which, when it is running on a computer or is loaded onto a computer, causes the computer to perform a method in accordance with any one of claims 1 to 17.

19. A computer program storage medium, comprising a program according to claim 18.

## Revendications

1. Procédé de planification assisté par ordinateur pour corriger des changements de forme d'os d'articulation, comportant les étapes suivantes consistant à :
- produire un ensemble de données d'imagerie tridimensionnelle, notamment un ensemble de données d'image volumique du voisinage de l'os d'articulation, en particulier au moyen d'un procédé d'imagerie médicale,
- identifier les éléments osseux de l'articulation (2, 3) dans l'ensemble de données et les déterminer en fonction de la forme, en particulier au moyen d'un procédé de segmentation d'image assisté par ordinateur,
- au moyen d'un traitement de données graphique, inscrire la partie de l'os d'articulation (1) à reconstruire avec une forme de base, en particulier géométrique, pouvant être attribuée,
- déterminer des déviations de l'os d'articulation (1) par rapport à la forme de base en établissant des distances de contour entre la forme de base et la forme de l'os d'articulation dans différents plans, un volume de déviation tridimensionnel (V) étant déterminé, et
- déterminer le volume de déviation (V) en vue de la planification d'une correction.

2. Procédé selon la revendication 1, dans lequel le procédé d'imagerie est un procédé de tomographie assistée par ordinateur, un procédé de tomographie par résonance magnétique nucléaire ou un procédé de radiographie incluant une détection volumétrique.

3. Procédé selon la revendication 1 ou 2, dans lequel le volume de déviation (V) est déterminé en comparant les contours dans des plans de coupe (E₂, Eₙ) tournés autour d'un axe (g), l'axe (g) étant un axe caractéristique de l'os d'articulation (1).

4. Procédé selon l'une quelconque des revendications 1 à 3, dans lequel le volume de déviation (V) est déterminé en comparant les contours dans des plans de coupe (E₂, Eₙ) adjacents les uns aux autres.

5. Procédé selon l'une quelconque des revendications 1 à 4, dans lequel la forme de base inclut une forme sphérique, une forme de selle, une forme cylindrique ou une combinaison de ces formes.

6. Procédé selon l'une quelconque des revendications 1 à 5, dans lequel la forme de base est une forme de base de l'os d'articulation (1), en particulier adaptée à la taille, provenant d'un atlas anatomique ou d'un autre modèle de comparaison, en particulier un modèle générique ou statistique.

7. Procédé selon l'une quelconque des revendications 1 à 6, dans lequel l'os d'articulation (1) est l'os du col fémoral (2) et la forme de base comporte une forme sphérique inscrite dans la tête de l'os fémoral.

8. Procédé selon la revendication 7, dans lequel les déviations de contour de la forme sphérique sont déterminées successivement dans plusieurs plans de coupe (E₁ à En) qui sont tournés autour de l'axe de col fémoral (g).

9. Procédé selon la revendication 8, dans lequel les déviations de contour dans un ou chaque plan de coupe (E₂, Eₙ) sont déterminées le long d'un rayon vecteur de la sphère qui prend une pluralité d'angles (α) par rapport à l'axe de col du col fémoral (2) qui varient sur l'intervalle de mesure.

10. Procédé selon la revendication 9, dans lequel l'intervalle de mesure par rapport à l'angle (α) vers l'extrémité libre de la tête fémorale débute à l'endroit où le contour de tête dévie pour la première fois par rapport à la forme de base.

11. Procédé selon la revendication. 9 ou 10, dans lequel l'intervalle de mesure par rapport à l'angle (α) vers le col fémoral se termine (αₙ) à l'endroit où l'angle (α) prend une valeur qui correspond à un angle réfléchi (β), dans lequel l'angle (β) est l'angle que prend un rayon vecteur sphérique par rapport à l'axe de col (g) lorsqu'il pointe vers la transition entre la tête d' os fémoral et le col fémoral (2), du côté opposé à la déviation de contour.

12. Procédé selon la revendication 9 ou 10, dans lequel l'intervalle de mesure par rapport à l'angle (α) vers le col fémoral (2) se termine (αₙ) à l'endroit où l'angle (α) prend une valeur standard qui est prédéfinie ou est calculée à partir d'un modèle générique ou statistique, dans lequel la valeur standard peut varier en fonction de l'orientation du plan de coupe (E₂, En).

13. Procédé selon l'une quelconque des revendications 1 à 12, dans lequel les données obtenues suivantes concernant le volume de déviation (V) ont été générées, de manière individuelle ou combinée, à partir d'un dispositif de traitement de données assisté par ordinateur en vue d'une utilisation ou d'un traitement supplémentaire :
- plans de coupe avec des déviations de contour et/ou une forme de base, en particulier le plan de coupe avec la plus grande déviation de contour,
- angles d'intervalle de mesure déterminés (α, β),
- caractéristiques osseuses telles qu'un centre de rotation d'articulation, une position d'axe de col, des plans pelviens, une position d'axe d'os fémoral.

14. Procédé selon la revendication 13, dans lequel les données sont générées sur un dispositif d'affichage, en particulier un écran.

15. Procédé selon la revendication 13 ou 14, dans lequel les données sont générées sur un système de navigation médical.

16. Procédé selon l'une quelconque des revendications 1 à 15, dans lequel un volume de reconstruction (V) est calculé à partir des volumes de déviation, en tenant compte d'une ou plusieurs des conditions limites suivantes :
- une profondeur d'os suffisante doit être maintenue,
- une nouvelle forme d'os d'articulation doit présenter des transitions plates et ne doit avoir aucune indentation ou des indentations minimales,
- une nouvelle forme d'os d'articulation doit se rapprocher le plus possible d'une forme naturelle d'os d'articulation (par comparaison avec des formes standard),
- l'influence des zones d'interférence entre les os est évaluée en fonction de leurs caractéristiques critiques,
- le volume de reconstruction, en particulier le volume à retirer, doit être le plus possible minimisé.

17. Procédé selon l'une quelconque des revendications 1 à 16, dans lequel une nouvelle forme d'os d'articulation est déterminée à l'aide du volume de déviation (V) ou du volume de reconstruction et une simulation de mouvement d'articulation assistée par ordinateur est réalisée avec la nouvelle forme d'os d'articulation sur la base d'une détection d'interférence, dans lequel l'agrandissement attendu de l'espace de liberté de mouvement est déterminé et notamment également généré.

18. Programme qui, lorsqu'il s'exécute sur un ordinateur ou est chargé dans un ordinateur, amène l'ordinateur à mettre en oeuvre un procédé selon l'une quelconque des revendications 1 à 17.

19. Support de mémorisation de programme informatique comportant un programme selon la revendication 18.
